(19) Europäisches Patentamt
European Patent Office
Office européen des brevets

(11) Veröffentlichungsnummer: **0 237 915 B1**

(12) # EUROPÄISCHE PATENTSCHRIFT

(45) Veröffentlichungstag der Patentschrift: **13.05.92**

(51) Int. Cl.$^5$: **C07D 487/04**, C09B 11/26, B41M 5/124, B41M 5/26, B41M 5/20

(21) Anmeldenummer: **87103388.2**

(22) Anmeldetag: **10.03.87**

(54) **Chromogene Phthalide, ihre Herstellung und Verwendung.**

(30) Priorität: **20.03.86 DE 3609344**

(43) Veröffentlichungstag der Anmeldung:
**23.09.87 Patentblatt 87/39**

(45) Bekanntmachung des Hinweises auf die Patenterteilung:
**13.05.92 Patentblatt 92/20**

(84) Benannte Vertragsstaaten:
**AT CH DE ES FR GB IT LI NL**

(56) Entgegenhaltungen:
**CH-A- 484 251**
**GB-A- 1 162 771**
**GB-A- 1 429 787**

(73) Patentinhaber: **BAYER AG**
**W-5090 Leverkusen 1 Bayerwerk(DE)**

(72) Erfinder: **Eckstein, Udo, Dr.**
**Wolfskaul 8**
**W-5000 Köln 80(DE)**

EP 0 237 915 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die GB-A 1 429 787 beschreibt Farbbildner, die eine wasserlöslich machende Gruppe enthalten.

Die GB-A 1 162 771 beschreibt Dialkylphthalide die Stickstoffatome in ungesättigten Fünfringen enthalten.

Die chromogenen Phthalide der vorliegenden Erfindung enthalten keine wasserlöslichen Gruppen, gleichzeitig befinden sich Stickstoffatome in ungesättigten Sechsringen.

Gegenstand der Erfindung sind chromogene Phthalide der allgemeinen Formel

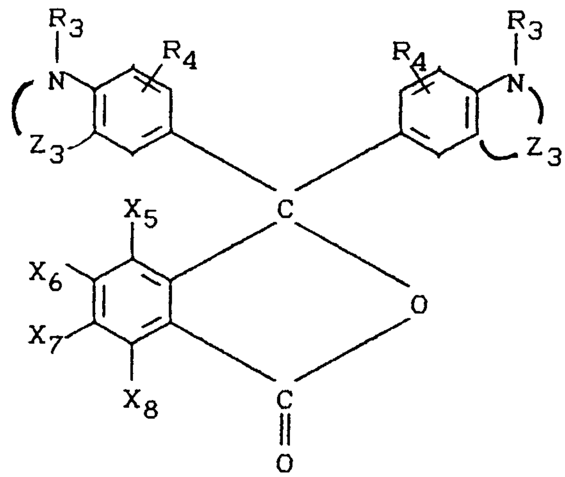

(II)

worin die beiden Reste

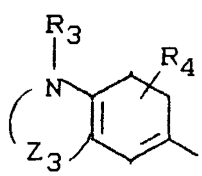

(1)

gleich oder verschieden sind und für

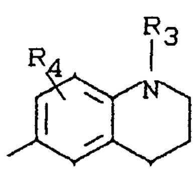

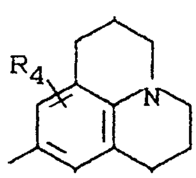

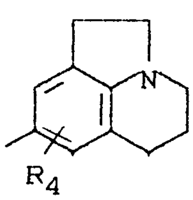

oder

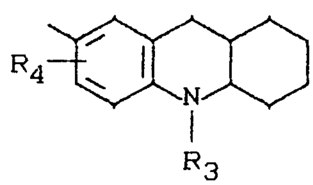

stehen, wobei die (teil)gesättigten Ringe bis zu 4 Reste aus der Gruppe Chlor, $C_1$-$C_4$-Alkyl, $C_1$-$C_4$-Alkoxy oder Phenyl tragen können, und wobei im Falle Tetrahydrochinolin und Julolidin die gesättigten Ringe mindestens einen dieser Reste tragen müssen,

$R_3$ Wasserstoff, gegebenenfalls durch Chlor, Hydroxy, $C_1$-$C_4$-Alkoxy oder Acetyloxy substituiertes $C_1$-$C_{12}$-Alkyl, Cyclohexyl, $C_1$-$C_4$-Alkylcarbonyl oder gegebenenfalls durch Chlor oder $C_1$-$C_4$-Alkyl substituiertes Benzyl oder Phenyl,

$R_4$ Wasserstoff, Chlor, $C_1$-$C_4$-Alkyl, $C_1$-$C_4$-Alkoxy, Amino, das durch 1 oder 2 gegebenenfalls durch Chlor, Cyano, $C_1$-$C_4$-Alkoxycarbonyl, Hydroxy oder $C_1$-$C_4$-Alkoxy substituierte $C_1$-$C_4$-Alkyl-, Cyclohexyl-, Phenyl- oder Benzylgruppen substituiert sein kann, und

$X_5$,$X_5$,$X_7$ und $X_5$ unabhängig voneinander Wasserstoff, Chlor, $C_1$-$C_4$-Alkyl, $C_1$-$C_4$-Alkoxy, gegebenenfalls durch Chlor, $C_1$-$C_4$-Alkyl oder $C_1$-$C_4$-Alkoxy substituiertes Phenyl, Benzyl oder Benzyloxy

bedeuten,
sowie deren Gemische und ihre Herstellung und ihre Verwendung in druckkopierfähigen und thermoreaktiven Aufzeichnungsmaterialien.

Beispiele für die Reste (1) sind:

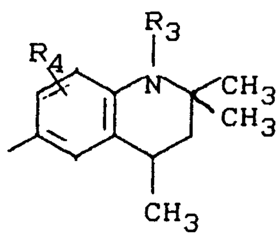

und

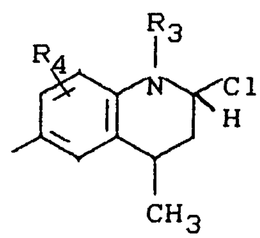

Besonders bevorzugte Verbindungen entsprechen der Formel

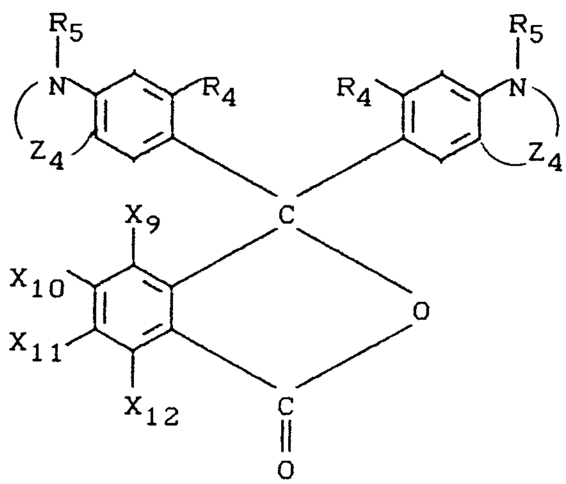

(III)

worin die beiden Reste

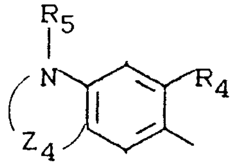

(2)

gleich oder verschieden sind und für

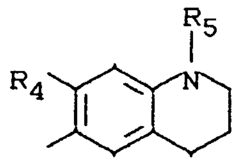

oder

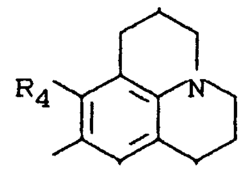

stehen, wobei $R_4$ die vorgenannte Bedeutung hat und die (teil)gesättigten Ringe bis zu 4 Reste aus der Gruppe Chlor, $C_1$-$C_4$-Alkyl, $C_1$-$C_4$-Alkoxy oder Phenyl tragen können und im Falle Indolin, Tetrahydrochinolin und Julolidin die gesättigten Ringe mindestens 1 dieser Reste tragen müssen,

$R_5$ Wasserstoff, gegebenenfalls durch Chlor, Hydroxy, Methoxy, Ethoxy oder Acetyloxy substituiertes $C_1$-$C_{12}$-Alkyl oder Benzyl,

$X_9$,$X_{10}$,$X_{11}$ und $X_{12}$ unabhängig voneinander Wasserstoff, Chlor, $C_1$-$C_4$-Alkyl, $C_1$-$C_4$-Alkoxy, Phenyl oder Benzyl bedeuten.

Von den Verbindungen (III) sind diejenigen hervorzuheben, deren Reste (2) gleich sind, Unter diesen Verbindungen entsprechen ganz besonders bevorzugte Verbindungen der Formel

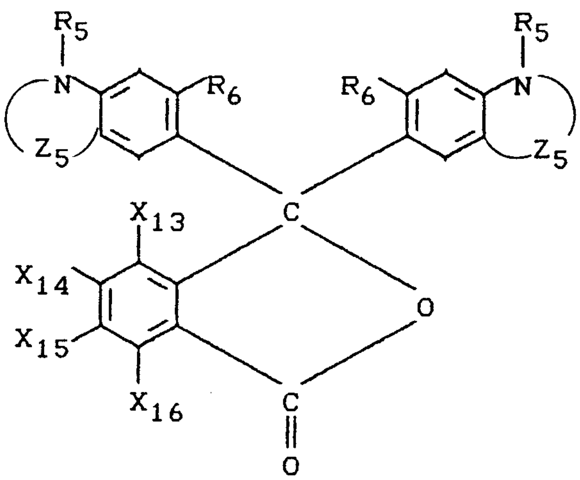

(IV)

worin die Reste

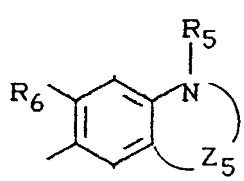

für

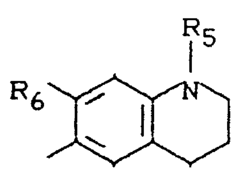

oder

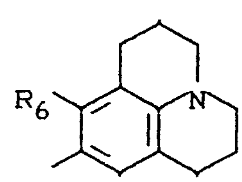

stehen, wobei

| | |
|---|---|
| $R_5$ | die vorstehend genannte Bedeutung hat |
| $R_6$ | Wasserstoff, Chlor, Methyl, Ethyl, Methoxy, Ethoxy, Amino, das durch 1 oder 2 gegebenenfalls durch Chlor, Cyano, Hydroxy, Methoxy oder Ethoxy substituierte $C_1$-$C_4$-Alkyl-Gruppen substituiert sein kann, |
| $X_{13}$,$X_{14}$,$X_{15}$ und $X_{16}$ | unabhängig voneinander Wasserstoff, Chlor, Methyl, Ethyl, Methoxy, Ethoxy oder Phenyl bedeuten, |

die (teil)hydrierten Ringe bis zu 4 Reste aus der Gruppe Chlor, Methyl, Ethyl, Methoxy, Ethoxy oder Phenyl tragen können und im Falle Indolin, Tetrahydrochinolin und Julolidin die gesättigten Ringe mindestens einen dieser Reste tragen müssen.

Weitere bevorzugte erfindungsgemäße Phthalide sind solche der Formel (IV), bei denen

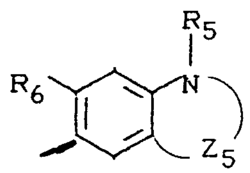

für

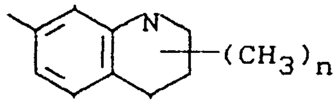

mit n = 1, 2 oder 3, insbesondere 3 steht.

Technisch besonders interessant sind auch die Gemische aus den unsymmetrischen und den beiden symmetrischen Verbindungen der Formel (III).

Ein weiterer Gegenstand der Erfindung ist ein Verfahren zur Herstellung der Phthalide der Formel (II), dadurch gekennzeichnet, daß man in einer Eintopfreaktion Anhydride der Formel

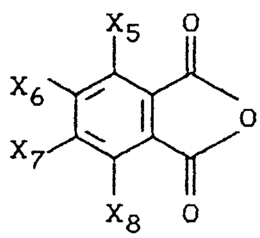

(V)

entweder gleichzeitig oder nacheinander mit Aminen der Formel

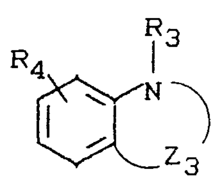

(VIa)

in einem für die Kondensation geeigneten Reaktionsmedium und in Gegenwart von Katalysatoren zu Ketocarbonsäuren der Formel

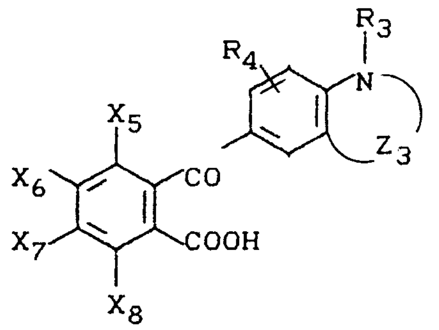

(VIIa)

kondensiert und diese ohne Zwischenisolierung unter Zugabe von wasserabspaltenden Kondensationsmitteln mit weiteren Aminen der Formel VIa zu Verbindungen der Formel (II), worin die Symbole die vorgenannte Bedeutung haben, umsetzt.

Auf diese Weise können symmetrische und unsymmetrische Verbindungen der Formel (II) und Gemische aus symmetrischen und unsymmetrischen Verbindungen der Formel (II) hergestellt werden.

Die erfindungsgemäßen Verbindungen der Formel (II) und ihre Gemische werden üblicherweise auch erhalten, wenn man die Ketocarbonsäuren der Formel (VIIa) isoliert und mit dem entsprechenden Amin (VIa) in Essigsäureanhydrid kondensiert.

Nach der vollständigen Umsetzung wird die Reaktionsmischung, evtl, auch nach Entfernen der inerten Lösungsmittel, auf beispielsweise Wasser oder ein niederes Alkanol ausgetragen. Durch Anheben des pH-Wertes mit beispielsweise Alkali- oder Erdalkalihydroxiden, Carbonaten, Hydrogencarbonaten, Ammoniak oder Aminen bis zum Verschwinden der Farbe der Mischung erhält man die Phthalide der Formel (II) oder ihre Mischungen, die entweder in üblicher Weise isoliert oder in der organischen Phase abgetrennt werden, Nach Entfernen des jeweiligen Lösungsmittels und Ausrühren in Alkanolen, beispielsweise Methanol, Ethanol, Propanol, Butanol, in Nitrilen, beispielsweise Acetonitril, in Ketonen, beispielsweise Aceton, 2-Butanon, in Estern, beispielsweise Essigsäureethylester, Essigsäurebutylester oder in Ethern, beispielsweise Diisopropylether, Di-sec.-butylether, erhält man die Phthalide der Formel (II) oder ihre Mischungen in hohen Ausbeuten und ausgezeichneter Qualität.

Geeignete Lösungsmittel sind aromatische Kohlenwasserstoffe, wie Toluol, Xylol, Chlorbenzol, o-Dichlorbenzol, Chlortoluol, oder Nitrobenzol, Halogenkohlenwasserstoffe wie Dichlorethan, Trichlorethylen, Methylenchlorid, Tetrachlorkohlenwasserstoff; Schwefelkohlenstoff oder Eisessig.

Geeignete Katalysatoren sind z.B. $AlCl_3$, $FeCl_3$, $SnCl_4$, $BF_3$, $TiCl_3$, $ZnCl_2$, $H_2SO_4$ oder $H_3PO_4$.

Geeignete wasserabspaltende Kondensationsmittel sind Alkansäureanhydride wie Essigsäureanhydrid oder Propionsäureanhydrid oder Trichloressigsäureanhydrid; Schwefelsäure, p-Toluolsulfonsäure, Polyphosphorsäure oder Phosphorpentoxid. Geeignete Reaktionstemperaturen liegen zwischen 20 und 180 ° C, bevorzugt sind 30 bis 120 ° C.

Die Phthalide der Formel (II) oder deren Mischungen sind normalerweise farblos oder höchstens schwach gefärbt.

Wenn diese Farbbilder mit einem vorzugsweise sauren Entwickler, d.h. einem Elektronenakzeptor in Kontakt gebracht werden, so ergeben sich intensive blaue, grünblaue, grüne, violette Farbtöne, die ausgezeichnet sublimations- und lichtecht sind.

Sie sind auch wertvoll im Gemisch mit einem oder mehreren anderen bekannten Farbbildnern, z.B. 3,3-Bis-(aminophenyl)-phthaliden,3,3-Bis-(indolyl)-phthaliden, 3-Amino-fluoranen, 2,6-Diamino-fluoranen, Leukoauraminen, Spiropyranen, Spirodipyranen, Chromenoindolen, Phenoxazinen, Phenothiazinen, Carbazolylmethanen oder anderen Triarylmethanleukofarbstoffen, um grüne, violette, blaue, marineblaue, graue oder schwarze Färbungen zu ergeben.

Die Phthalide der Formel (II) oder deren Mischungen zeigen sowohl auf phenolischen Unterlagen wie auch auf Salicylat-Unterlagen und besonders auf aktivierten Tonen eine gute Farbintensität und Lichtechtheit. Sie eignen sich vor allem als Farbbildner für die Verwendung in einem wärmeempfindlichen oder druckempfindlichen Aufzeichnungsmaterial, das sowohl Kopier- als auch Registriermaterial sein kann. Ihre Entwicklungsgeschwindigkeit ist in Abhängigkeit von den Substituenten unterschiedlich. Im allgemeinen zeichnen sie sich jedoch durch eine hohe Entwicklungsgeschwindigkeit aus bei gleichzeitig reduzierter Empfindlichkeit der Aufzeichnungsmaterialien gegenüber unbeabsichtigter vorzeitiger Entwicklung.

Typische Beispiele für Entwickler in druckempfindlichen Materialien sind anorganische Stoffe wie Tone, Metallsalze oder -oxide oder organische Polymerisate wie Phenolharze.

Die Verbindungen der Formel (II) oder deren Mischungen werden vorzugsweise auch als Farbbildner in einem thermoreaktiven Aufzeichnungsmaterial verwendet.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Meßinstrumenten wie Elektrokardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Als Entwickler eignen sich die gleichen Elektronenakzeptoren wie sie in druckempfindlichen Papieren verwendet werden, vorzugsweise phenolische Verbindungen, die beispielsweise in der DE-PS 1 251 348 beschrieben sind, sowie Borsäure und organische, vorzugsweise aliphatische Dicarbonsäuren.

Vorzugsweise werden zur Herstellung des thermoreaktiven Aufzeichnungsmaterials schmelzbare, filmbildende, in Wasser lösliche oder quellbare Bindemittel verwendet. Bei Einwirkung von Wärme erweicht oder schmilzt das Bindemittel, so daß der Farbbildner mit dem Entwickler in Kontakt kommt und sich eine Farbe bilden kann. Geeignete Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine und Stärke.

Die beschriebenen Verfahren und Zubereitungen sind beispielsweise aus den US-Patentschriften 2 800 457, 2 800 458, 2 946 753, 3 096 189 und 3 193 404 und aus den deutschen Offenlegungsschriften 2 555 080 und 2 700 937 bekannt.

Die Phthalide der Formel (II) bzw. die hieraus durch Ringöffnung gebildeten Farbstoffe sind zum Anfärben von Polyacrylnitril, tannierter Baumwolle und anderen sauer modifizierten Fasern, Geweben und Pulvern geeignet.

Beispiel 1

Zu 14,8 g (0,1 Mol) Phthalsäureanhydrid und 24,4 g (0,12 Mol) 1-Ethyl-2,2,4-trimethyl-1,2,3,4-tetrahydrochinolin in 100 ml wasserfreiem Chlorbenzol gibt man portionsweise 13,7 g (0,1 Mol) gepulvertes Aluminiumchlorid (98 %ig), so daß die Temperatur 30° C nicht übersteigt. Man verrührt 1 Stunde bei 30° C und 1 Stunde bei 60° C. Danach wird die Reaktionsmischung einer Wasserdampfdestillation unterzogen. Abkühlen und Absaugen liefert 20,5 g (58,4 % der Theorie) Rohprodukt vom Schmp.: 213-218° C der Ketocarbonsäure der Formel

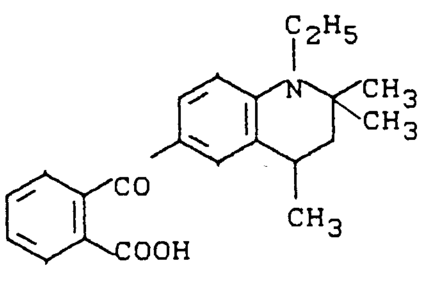

(1)

die aus 70 %iger Essigsäure umgelöst werden kann; gelbliche Kristalle vom Schmp.: 221-23° C.

10,5 g (0,03 Mol) der Ketocarbonsäure der Formel (1) und 6,1 g (0,03 Mol) 1-Ethyl-2,2,4-trimethyl-1,2,3,4-tetrahydrochinolin werden in 50 ml Essigsäureanhydrid 2 Stunden bei 100° C verrührt. Nach dem Erkalten werden 50 ml Ethanol hinzugefügt, die Lösung auf 250 ml 10 %ige Natronlauge ausgetragen und 1

Stunde verrührt Es wird abgesaugt, intensiv mit Ethanol gewaschen und getrocknet: 14,1 g (87,6 % der Theorie) farbloses Pulver vom Schmp.: 118 bis 123° C der Formel

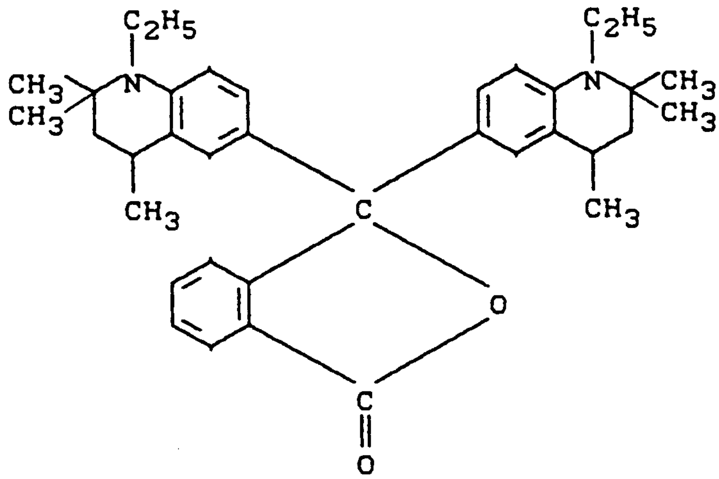

(2)

aus Hexan farblose Kristalle vom Schmp.: 131-34° C

| | | | | |
|---|---|---|---|---|
| $C_{36}H_{44}N_2O_2$ (536,7) | Ber.:<br>Gef.: | 80,55 % C,<br>80,5 % C, | 8,3 % H,<br>8,5 % H, | 5,2 % N<br>5,0 % N |

Eine Lösung in Eisessig wird grün mit $\lambda_{max}$ = 652 nm und $\lambda_2$ = 420 nm.
Auf Säureton oder Bisphenol A wird eine kräftige grüne Farbe mit hohen Echtheiten entwickelt.

Beispiel 2

22,3 g (0,11 Mol) 1-Ethyl-2,2,4-trimethyl-1,2,3,4-tetrahydrochinolin und 7,4 g (0,05 Mol) Phthalsäureanhydrid werden in 100 ml wasserfreiem Chlorbenzol gelöst und bei Raumtemperatur portionsweise mit 7 g (0,05 Mol) Aluminiumchlorid (95 %ig) versetzt. Man hält die Raumtemperatur durch Außenkühlung. Es wird dann 4 Stunden bei 60° C nachgerührt. Nach der Zugabe von 30 g Essigsäureanhydrid werden weitere 4 Stunden bei dieser Temperatur verrührt. Die Reaktionsmischung wird auf 500 ml Eiswasser ausgetragen und die Chlorbenzolphase abgetrennt. Diese wird mit 100 ml 10 %iger Natronlauge entfärbt, mit 100 ml Wasser ausgeschüttelt und abgetrennt. Nach Entfernen des Lösungsmittels im Vakuum gibt man 80 ml Methanol zum öligen Rückstand, rührt 1 Stunde, saugt ab, wäscht mit Methanol und trocknet. 24,2 g (90,3 % der Theorie) farblose Kristalle vom Schmp.: 134-36° C der Formel (2).

Beispiel 3

Ersetzt man im obigen Beispiel 1-Ethyl-2,2,4-trimethyl-1,2,3,4-tetrahydrochinolin durch die entsprechende Menge 1,2,2,4-Tetramethyl-1,2,3,4-tetrahydrochinolin und verfährt wie angegeben, so erhält man 19,8 g (78 % der Theorie) farblose Kristalle der Verbindung der Formel

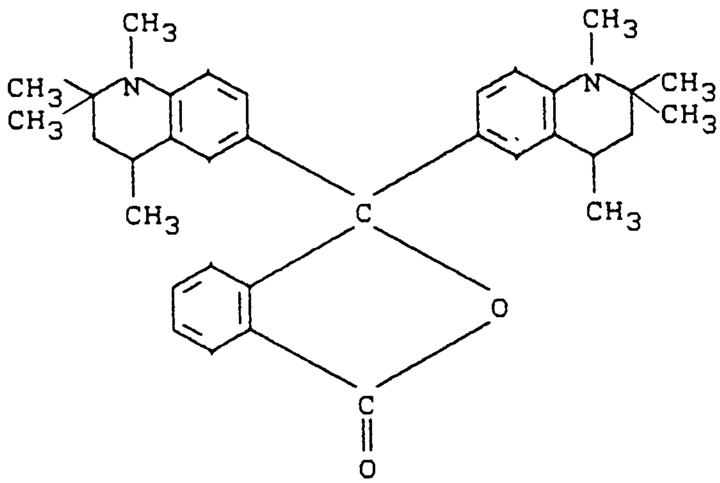

(3)

mit einem Schmp.: 141-45° C, die auf Säureton, Bisphenol A und Salicylat eine kräftige grüne Farbe entwickelt. Absorptionsspektrum in Eisessig: $\lambda_{max}$ = 646 nm und $\lambda_2$ = 420 nm.

Analog den Beispielen 2 und 3 werden folgende Phthalide der Formel

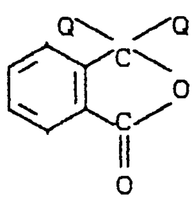

hergestellt.

| Formel Nr. | Q | Farbton auf Säureton oder Bisphenol A | $\lambda_{max}$ in Eisessig |
|---|---|---|---|
| 4 | 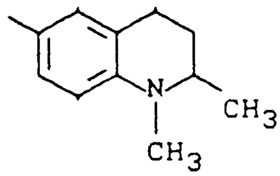 | grün | 644 nm |
| 5 | 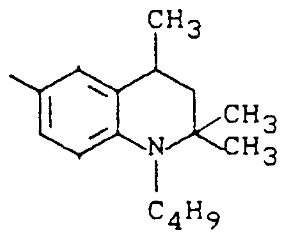 | grün | 652 nm |
| 6 | 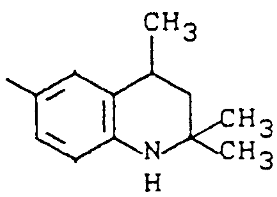 | blau grün | 621 nm |
| 7 | 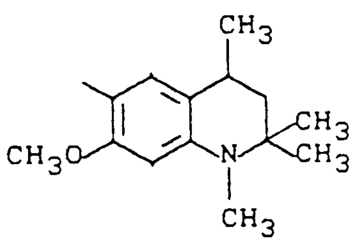 | blau grün | 660 nm |
| 8. | 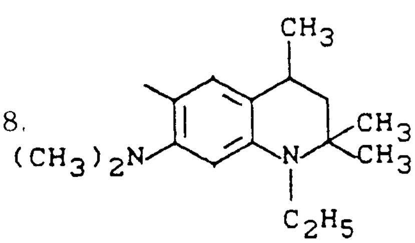 | blau | 635 nm |

| Formel Nr. | Q | Farbton auf Säureton oder Bisphenol A | $\lambda_{max}$ in Eisessig |
|---|---|---|---|
| 9 | 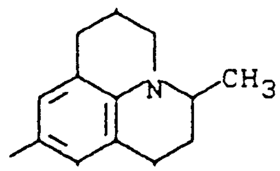 | dunkelgrün | 668 nm |

Beispiel 4

Zur Lösung von 20,3 g (0,1 Mol) 1-Ethyl-2,2,4-trimethyl-1,2,3,4-tetrahydrochinolin und 14,3 g (0,05 Mol) Tetrachlorphthalsäureanhydrid in 100 ml wasserfreiem 1,2-Dichlorbenzol werden portionsweise 7 g (0,05 Mol) Aluminiumchlorid (95 %ig) hinzugefügt. Dabei hält man die Temperatur bei 25° C, rührt 1 Stunde bei dieser Temperatur. Nach 2 Stunden bei 60° C werden 30 g Essigsäureanhydrid zugetropft und weitere 2 Stunden bei 60° C gerührt. Die Reaktionsmischung wird mit 300 ml 1,2-Dichlorbenzol verdünnt, auf 700 ml Eiswasser ausgetragen und abgetrennt. Die organische Phase wird mit zweimal 100 ml 10 %iger Natronlauge und 100 ml Wasser ausgeschüttelt und abgetrennt. Nach Entfernen des Lösungsmittels im Vakuum wird der Rückstand mit 100 ml Methanol verrührt, abgesaugt, mit Methanol gewaschen und getrocknet. Man erhält 33 g (98 % der Theorie) gelbe Kristalle vom Schmp.: 220-25° C und der Formel

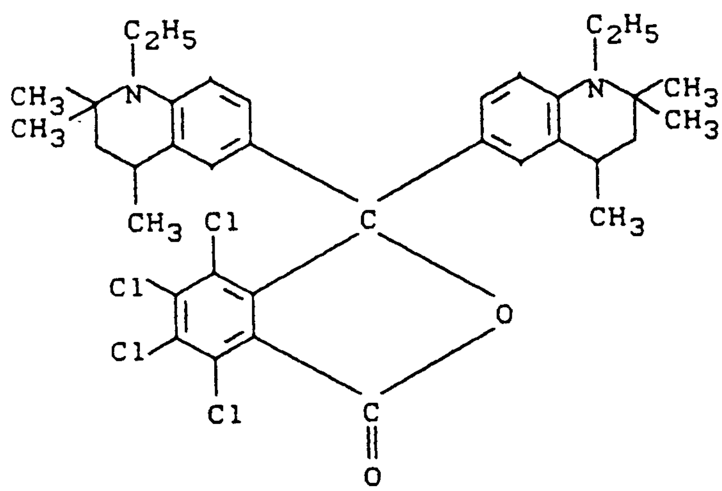

(10)

die aus Methylcyclohexan umkristallisiert werden. Schmp.: 227-29° C.
Auf Säureton und Bisphenol A erhält man kräftige grüne Farbtöne.

Wie im Beispiel 4 beschrieben erhalt man auch die Phthalide der Formel

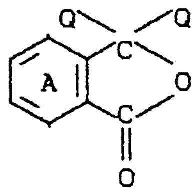

| Formel Nr. | Substituenten von Ring A | Q | Farbton auf Säureton oder Bisphenol A | $\lambda_{max}$ in Eisessig |
|---|---|---|---|---|
| 11 | Isomerengemisch aus 5-$OCH_3$ und 6-$OCH_3$ | 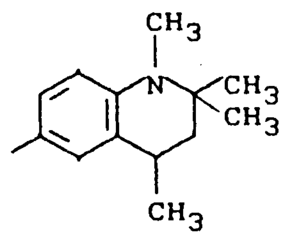 | blaugrün | 645 nm |
| 12 | 5-$OCH_3$ 6-$OCH_3$ | 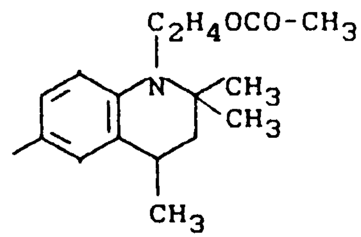 | blaugrün | 648 nm |
| 13 | 5-$OCH_3$ 6-$OCH_3$ | 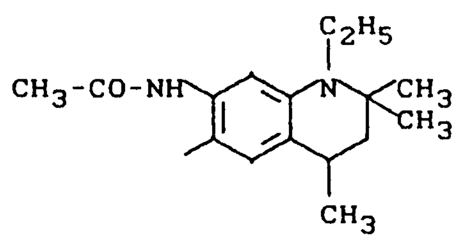 | schwarzgrün | 720 nm |

| Formel Nr. | Substituenten von Ring A | Q | Farbton auf Säureton oder Bisphenol A | $\lambda_{max}$ in Eisessig |
|---|---|---|---|---|
| 14 | 5-$CH_3$ 6-$CH_3$ | 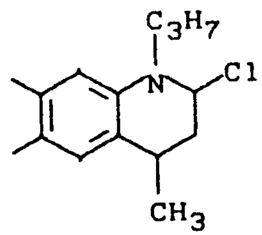 | dunkelgrün | 665 nm |

## Patentansprüche

**1.** Chromogene Phthalide der Formel

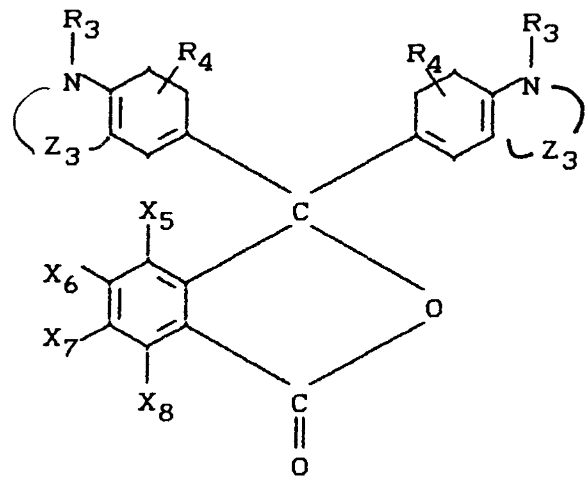

worin die beiden Reste

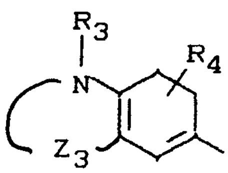

gleich oder verschieden sind und für

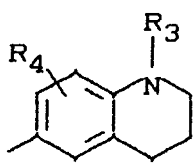

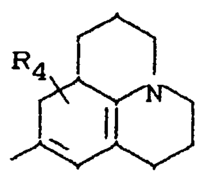,

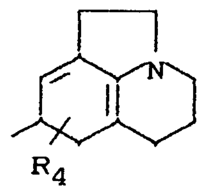

oder

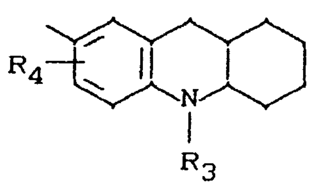

stehen, wobei die (teil)gesättigten Ringe bis zu 4 Reste aus der Gruppe Chlor, $C_1$-$C_4$-Alkyl, $C_1$-$C_4$-Alkoxy oder Phenyl tragen können, und wobei im Falle Tetrahydrochinolin und Julolidin die gesättigten Ringe mindestens einen dieser Reste tragen müssen,

$R_3$ Wasserstoff, gegebenenfalls durch Chlor, Hydroxy, $C_1$-$C_4$-Alkoxy oder Acetyloxy substituiertes $C_1$-$C_{12}$-Alkyl, Cyclohexyl, $C_1$-$C_4$-Alkylcarbonyl oder gegebenenfalls durch Chlor oder $C_1$-$C_4$-Alkyl substituiertes Benzyl oder Phenyl,

$R_4$ Wasserstoff, Chlor, $C_1$-$C_4$-Alkyl, $C_1$-$C_4$-Alkoxy, Amino, das durch 1 oder 2 gegebenenfalls durch Chlor, Cyano, $C_1$-$C_4$-Alkoxycarbonyl, Hydroxy oder $C_1$-$C_4$-Alkoxy substituierte $C_1$-$C_4$-Alkyl-, Cyclohexyl-, Phenyl- oder Benzylgruppen

substituiert sein kann, und

$X_5$,$X_5$,$X_7$ und $X_5$ unabhängig voneinander Wasserstoff, Chlor, $C_1$-$C_4$-Alkyl, $C_1$-$C_4$-Alkoxy, gegebenenfalls durch Chlor, $C_1$-$C_4$-Alkyl oder $C_1$-$C_4$-Alkoxy substituiertes Phenyl, Benzyl oder Benzyloxy

bedeuten.

**2.** Chromogene Phthalide gemäß Anspruch 1 der Formel

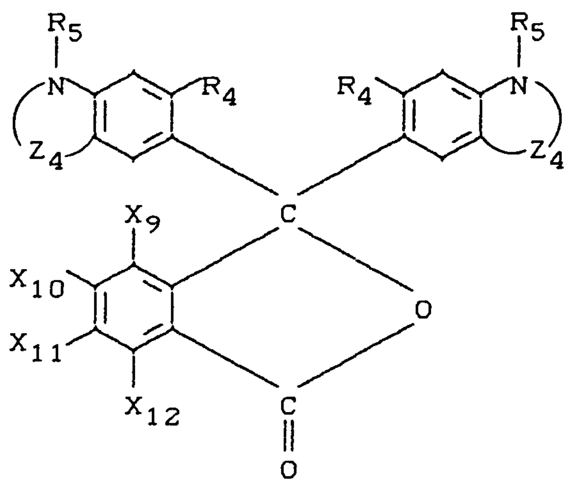

worin die beiden Reste

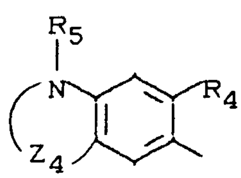

gleich oder verschieden sind und für

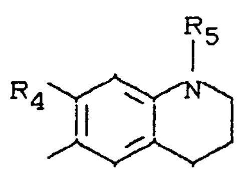

oder

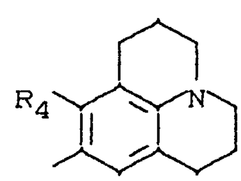

stehen, wobei $R_4$ die in Anspruch 1 angegebene Bedeutung hat und die (teil)gesättigten Ringe bis zu 4 Reste aus der Gruppe Chlor, $C_1$-$C_4$-Alkyl, $C_1$-$C_4$-Alkoxy oder Phenyl tragen können, und die gesättigten Ringe mindestens einen dieser Reste tragen müssen,

$R_5$ Wasserstoff, gegebenenfalls durch Chlor, Hydroxy, Methoxy, Ethoxy oder Acetyloxy substituiertes $C_1$-$C_{12}$-Alkyl oder Benzyl

$X_9$,$X_{10}$,$X_{11}$ und $X_{12}$ unabhängig voneinander Wasserstoff, Chlor, $C_1$-$C_4$-Alkyl, $C_1$-$C_4$-Alkoxy, Phenyl oder Benzyl bedeuten.

**3.** Chromogene Phthalide gemäß Ansprüchen 1 und 2 der Formel

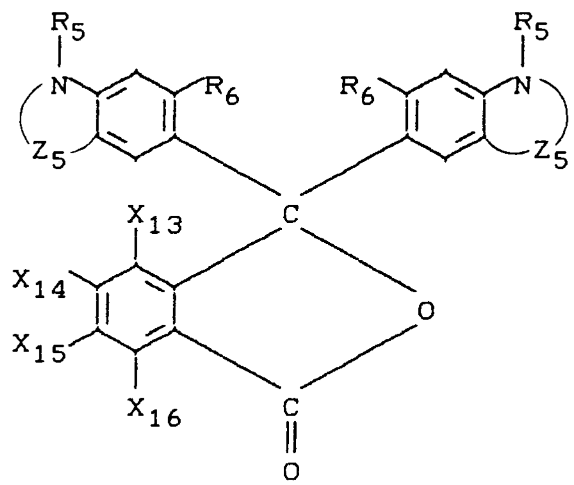

worin die Reste

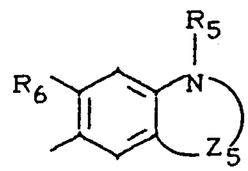

für

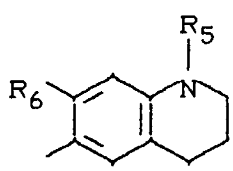

oder

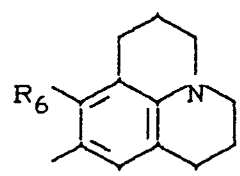

stehen, wobei

$R_5$ die in Anspruch 2 angegebene Bedeutung hat,

$R_6$ Wasserstoff, Chlor, Methyl, Ethyl, Methoxy, Ethoxy, Amino, das durch 1 oder 2 gegebenenfalls durch Chlor, Cyano, Hydroxy, Methoxy oder Ethoxy substituierte $C_1$-$C_4$-Alkyl-Gruppen substituiert sein kann,

$X_{13}$,$X_{14}$,$X_{15}$ und $X_{16}$ unabhängig voneinander Wasserstoff, Chlor, Methyl, Ethyl, Methoxy, Ethoxy oder Phenyl bedeuten,

die (teil)hydrierten Ringe durch bis zu 4 Reste aus der Gruppe Chlor, Methyl, Ethyl, Methoxy, Ethoxy oder Phenyl tragen können und mindestens einen dieser Reste tragen müssen.

**4.** Chromogene Phthalide der Formel des Anspruchs 3, worin

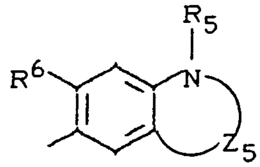

für

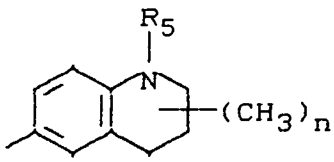

mit n = 1, 2 oder 3, insbesondere 3, steht.

**5.** Mischungen der Phthalide gemäß Anspruch 2 aus symmetrischen und unsymmetrischen Verbindungen der Formel von Anspruch 2.

**6.** Verfahren zur Herstellung der Phthalide des Anspruchs 1, dadurch gekennzeichnet, daß man in einer Eintopfreaktion Anhydride der Formel

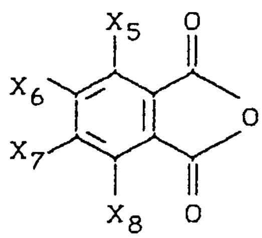

entweder gleichzeitig oder nacheinander mit Aminen der Formel

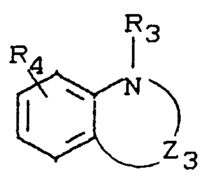

in einem für die Kondensation geeigneten Reaktionsmedium und in Gegenwart von Katalysatoren zur Ketocarbonsäuren der Formel

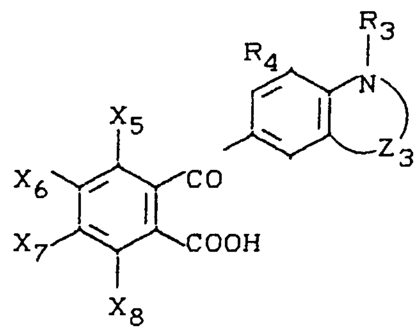

worin die Symbole die in Anspruch 1 angegebene Bedeutung haben, kondensiert und diese ohne Zwischenisolierung unter Zugabe von wasserabspaltenden Kondensationsmitteln mit weiteren Aminen der obengenannten Formel oder ihren Mischungen umsetzt.

**7.** Verwendung eines Phthalids der im Anspruch 1 angegebenen Formel als Farbbildner in druck-, wärme- und elektrosensitivem Aufzeichnungsmaterial.

**8.** Druck-, warme- und elektrosensitives Aufzeichnungsmaterial, dadurch gekennzeichnet, daß es in seinem Reaktantensystem mindestens ein Phthalid der in Anspruch 1 angegebenen Formel als Farbbildner enthält.

**Claims**

**1.** Chromogenic phthalides of the formula

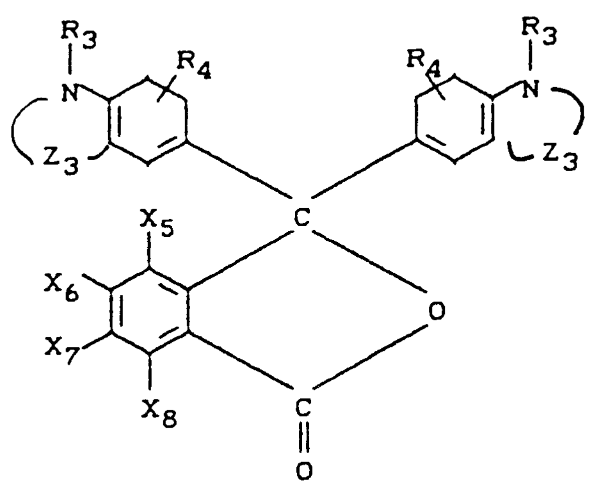

wherein the two radicals

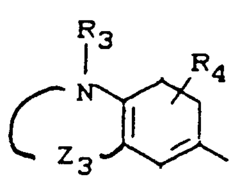

are identical or different and represent

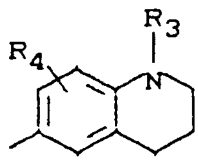

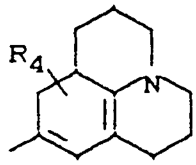

,

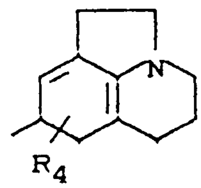

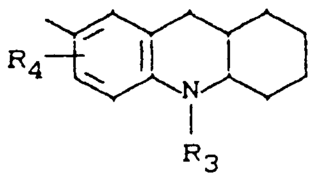

where the (partially) saturated rings can carry up to 4 radicals from the group comprising chlorine, $C_1$-$C_4$-alkyl, $C_1$-$C_4$-alkoxy or phenyl and, in the case of tetrahydroquinoline and julolidine, the saturated rings must carry at least one of these radicals,

$R_3$ denotes hydrogen, $C_1$-$C_{12}$-alkyl which is optionally substituted by chlorine, hydroxyl, $C_1$-$C_4$-alkoxy or acetyloxy, cyclohexyl, $C_1$-$C_4$-alkylcarbonyl, or benzyl or phenyl which are optionally substituted by chlorine or $C_1$-$C_4$-alkyl,

$R_4$ denotes hydrogen, chlorine, $C_1$-$C_4$-alkyl, $C_1$-$C_4$-alkoxy, amino which can be substituted by 1 or 2 $C_1$-$C_4$-alkyl, cyclohexyl, phenyl or benzyl groups which are optionally substituted by chlorine, cyano, $C_1$-$C_4$-alkoxycarbonyl, hydroxyl or $C_1$-$C_4$-alkoxy, and

$X_5$, $X_5$, $X_7$ and $X_5$ independently of one another denote hydrogen, chlorine, $C_1$-$C_4$-alkyl, $C_1$-$C_4$-alkoxy, or phenyl, benzyl or benzyloxy which are optionally substituted by chlorine, $C_1$-$C_4$-alkyl or $C_1$-$C_4$-alkoxy.

**2.** Chromogenic phthalides according to Claim 1 of the formula

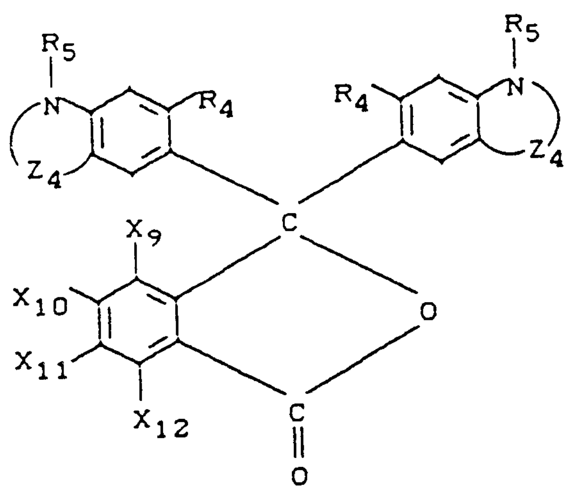

wherein the two radicals

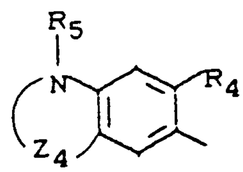

are identical or different and represent

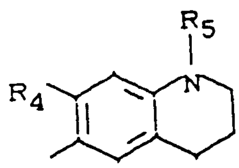

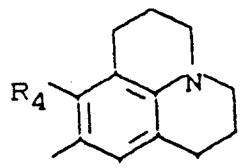

where $R_4$ has the meaning given in Claim 1 and the (partially) saturated rings carry up to 4 radicals from the group comprising chlorine, $C_1$-$C_4$-alkyl, $C_1$-$C_4$-alkoxy or phenyl and the saturated rings must carry at least one of these radicals,

$R_5$ denotes hydrogen, or $C_1$-$C_{12}$-alkyl or benzyl which are optionally substituted by chlorine, hydroxyl, methoxy, ethoxy or acetyloxy, and

$X_9$, $X_{10}$, $X_{11}$ and $X_{12}$ independently of one another denote hydrogen, chlorine, $C_1$-$C_4$-alkyl, $C_1$-$C_4$-alkoxy, phenyl or benzyl.

**3.** Chromogenic phthalides according to Claims 1 and 2, of the formula

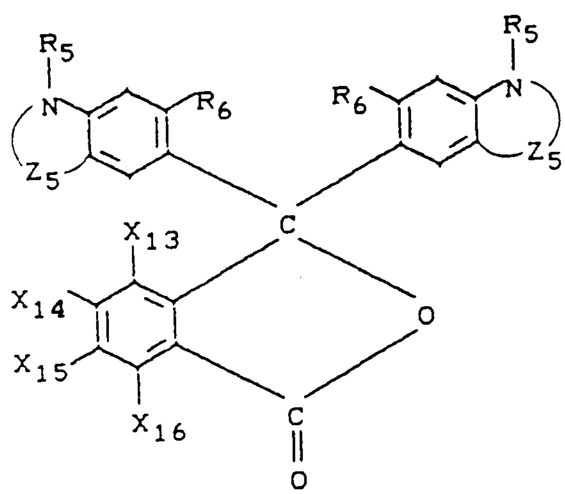

wherein the radicals

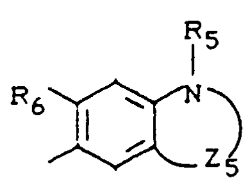

represent

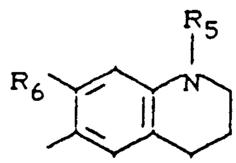

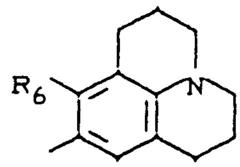

where

$R_5$ has the meaning given in Claim 2,

$R_6$ is hydrogen, chlorine, methyl, ethyl, methoxy, ethoxy or amino which can be substituted by 1 or 2 $C_1$-$C_4$-alkyl groups which are optionally substituted by chlorine, cyano, hydroxyl, methoxy or ethoxy,

$X_{13}$, $X_{14}$, $X_{15}$ and $X_{16}$ independently of one another denote hydrogen, chlorine, methyl, ethyl, methoxy, ethoxy or phenyl,

the (partially) hydrogenated rings can carry up to 4 radicals from the group comprising chlorine, methyl, ethyl, methoxy, ethoxy or phenyl and must carry at least one of these radicals.

**4.** Chromogenic phthalides of the formula of Claim 3, wherein

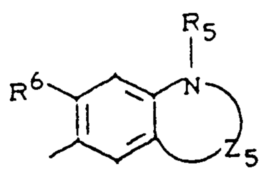

represents

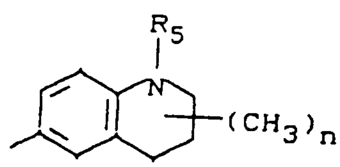

with n = 1, 2 or 3, in particular 3.

**5.** Mixtures of the phthalides according to Claim 2 consisting of symmetrical and unsymmetrical compounds of the formula of Claim 2.

**6.** Process for preparing the phthalides of Claim 1, characterised in that, in a one-pot reaction, anhydrides of the formula

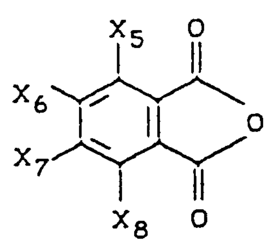

are condensed either simultaneously or successively with amines of the formula

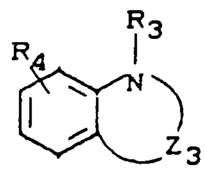

in a reaction medium suitable for the condensation and in the presence of catalysts to give ketocarboxylic acids of the formula

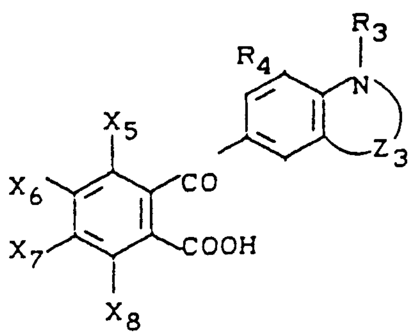

wherein the symbols have the meaning indicated in Claim 1, and these ketocarboxylic acids are reacted without intermediate isolation and with addition of dehydrating condensing agents with further amines of the abovementioned formula or their mixtures.

**7.** Use of a phthalide of the formula given in Claim 1 as a colour former in pressure-sensitive, heat-sensitive and electro-sensitive recording material.

**8.** Pressure-sensitive, heat-sensitive and electro-sensitive recording material, characterised in that, in its reactant system, it contains at least one phthalide of the formula indicated in Claim 1 as the colour former.

**Revendications**

**1.** Phtalides chromogènes de formule

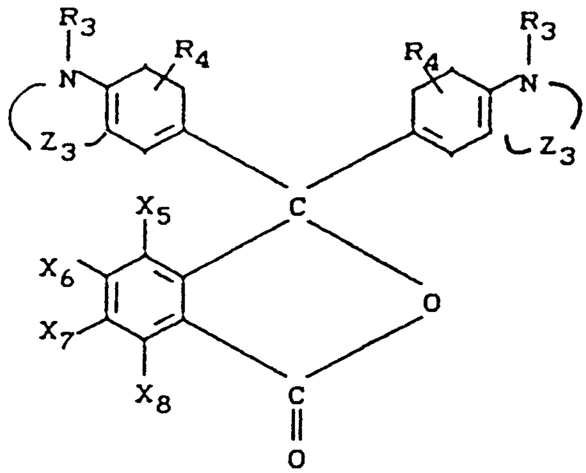

dans laquelle tes deux groupes

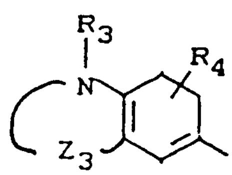

identiques ou différents, sont des groupes

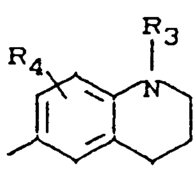

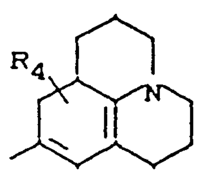

,

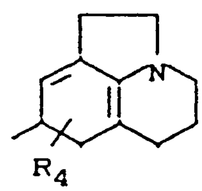

ou

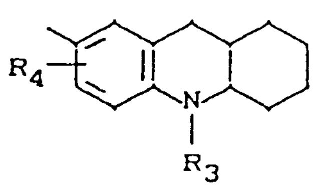

les cycles (partiellement) saturés pouvant porter jusqu'à 4 substituants choisis parmi le chlore, les groupes alkyle en $C_1$-$C_4$, alcoxy en $C_1$-$C_4$ ou phényle et, dans le cas de la tétrahydroquinoléine et de la julolidine, les cycles saturés doivent porter au moins un de ces substituants,

$R_3$ représente l'hydrogène, un groupe alkyle en $C_1$-$C_{12}$ éventuellement substitué par le chlore, des groupes hydroxy, alcoxy en $C_1$-$C_4$ ou acétyloxy, un groupe cyclohexyle, (alkyle en $C_1$-$C_4$)-carbonyle ou un groupe benzyle ou phényle éventuellement substitué par le chlore ou un groupe alkyle en $C_1$-$C_4$,

$R_4$ représente l'hydrogène, le chlore, un groupe alkyle en $C_1$-$C_4$, alcoxy en $C_1$-$C_4$, un groupe amino qui peut être substitué par un ou deux groupes alkyle en $C_1$-

$C_4$, cyclohexyle, phényle ou benzyle eux-mêmes éventuellement substitués par le chlore, des groupes cyano, (alcoxy en $C_1$-$C_4$)-carbonyle, hydroxy ou alcoxy en $C_1$-$C_6$, et

$X_5$, $X_5$, $X_7$ et $X_5$ représentent chacun, indépendamment les uns des autres, l'hydrogène, le chlore, un groupe alkyle en $C_1$-$C_4$,alcoxy en $C_1$-$C_4$, un groupe phényle, benzyle ou benzyloxy éventuellement substitué par le chlore, des groupes alkyle en $C_1$-$C_4$ ou alcoxy en $C_1$-$C_4$.

**2.** Phtalides chromogènes selon la revendication 1, de formule

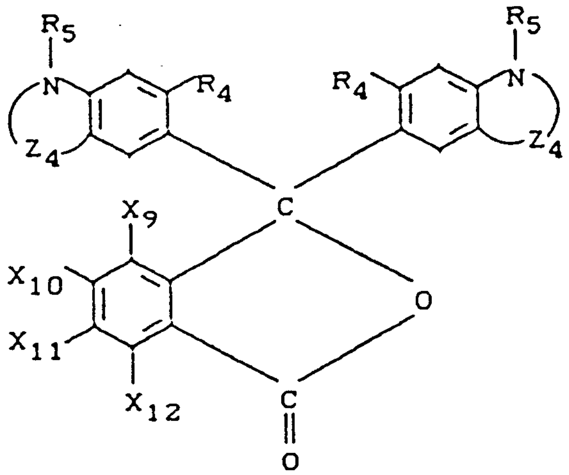

dans laquelle les deux groupes

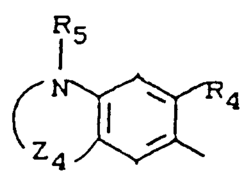

identiques ou différents, sont des groupes

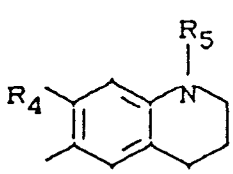

ou

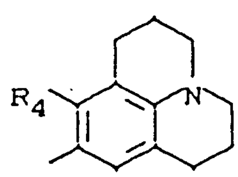

$R_4$ ayant les significations indiquées dans la revendication 1 et les cycles (partiellement) saturés pouvant porter jusqu'à 4 substituants choisis parmi le chlore, les groupes alkyle en $C_1$-$C_4$, alcoxy en $C_1$-$C_4$ ou phényle, et les cycles saturés doivent porter au moins un de ces substituants,

$R_5$ représente l'hydrogène, un groupe alkyle en $C_1$-$C_{12}$ éventuellement substitué par le chlore, des groupes hydroxy, méthoxy, éthoxy ou acétyloxy ou un groupe benzyle,

$X_9$, $X_{10}$, $X_{11}$ et $X_{12}$ représentent chacun, indépendamment les uns des autres, l'hydrogène, le chlore, un groupe alkyle en $C_1$-$C_4$,alcoxy en $C_1$-$C_4$, phényle ou benzyle.

**3.** Phtalides chromogènes selon les revendications 1 et 2, de formule

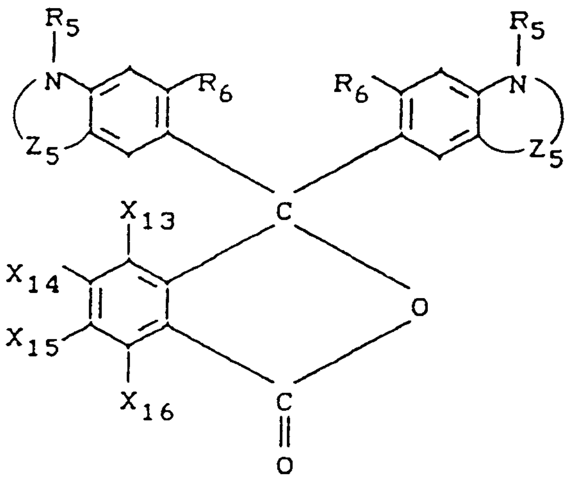

dans laquelle les groupes

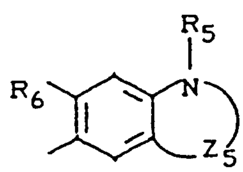

sont des groupes

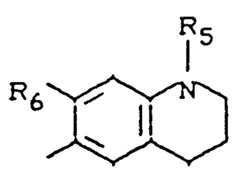

ou

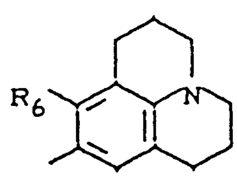

| | |
|---|---|
| $R_5$ | ayant les significations indiquées dans la revendication 2, |
| $R_6$ | représente l'hydrogène, le chlore, un groupe méthyle, éthyle, méthoxy, éthoxy, un groupe amino qui peut être substitué par un ou deux groupes alkyle en $C_1$-$C_4$ eux-mêmes éventuellement substitués par le chlore, des groupes cyano, hydroxy, méthoxy ou éthoxy, |
| $X_{13}$, $X_{14}$, $X_{15}$ et $X_{16}$ | représentent chacun, indépendamment les uns des autres, l'hydrogène, le chlore, un groupe méthyle, éthyle, méthoxy, éthoxy ou phényle, |

les cycles (partiellement) hydrogénés pouvant porter jusqu'à 4 substituants choisis parmi le chlore, les groupes méthyle, éthyle, méthoxy, éthoxy ou phényle et doivent porter au moins un de ces substituants.

**4.** Phtalides chromogènes répondant à la formule de la revendication 3 dans laquelle

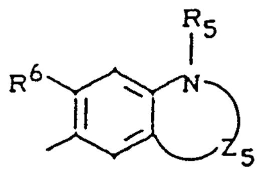

représente

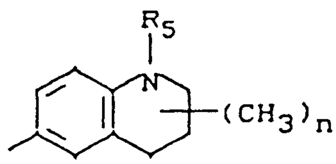

n étant égal à 1, 2 ou 3 et tout spécialement à 3.

**5.** Mélanges des phtalides selon la revendication 2, consistant en composés symétriques et asymétriques répondant à la formule de la revendication 2.

**6.** Procédé de préparation des phtalides de la revendication 1, caractérisé en ce que, dans une réaction en un seul récipient, on condense des anhydrides de formule

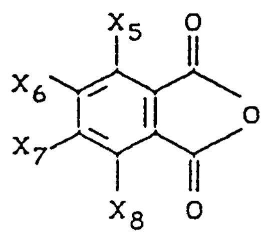

simultanément ou successivement, avec des amines de formule

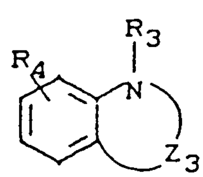

dans un milieu de réaction approprié à la condensation et en présence de catalyseurs, la réaction donnant des acides cétocarboxyliques de formule

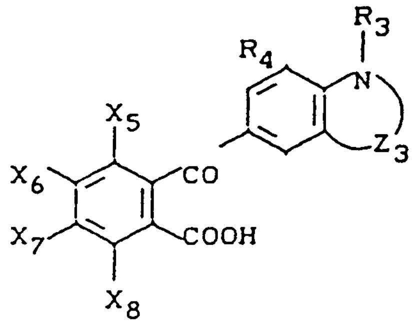

dans laquelle les symboles ont les significations indiquées dans la revendication 1, qu'on fait réagir, sans les isoler et avec adjonction d'agents de condensation déshydratants, avec d'autres amines de formule ci-dessus ou leurs mélanges.

**7.** Utilisation d'un phtalide répondant à la formule de la revendication 1 en tant qu'agent chromogène dans des matériaux d'enregistrement sensibles à la pression, thermosensibles et électrosensibles.

**8.** Matériaux d'enregistrement sensibles à la pression, thermosensibles et électrosensibles, caractérisés en ce qu'ils contiennent dans leur système de réactifs, en tant que chromogène, au moins un phtalide répondant à la formule de la revendication 1.